# EUROPEAN PATENT APPLICATION

(11) **EP 4 186 983 A1**
(43) Date of publication of application: **31.05.2023**
(21) Application number: 21880451.6
(22) Date of filing: 12.10.2021
(51) Int. Cl.: C12Q 1/6883, G01N 33/68, G01N 33/53

(54) **BIOMARKER COMPOSITION FOR PREDICTING PROGNOSIS OF BRAIN DISEASES CAUSED BY MICROPLASTIC EXPOSURE AND METHOD FOR PREDICTING PROGNOSIS USING SAME**

(30) Priority: 14.10.2020 KR 20200132403
(71) Applicant: Korea Institute of Radiological & Medical Sciences, Seoul 01812 (KR)
(72) Inventor: KIM, Jin Su, Seoul 01816 (KR); KIM, Hyeongi, Seoul 02007 (KR); ZAHEER, Javeria, Seoul 01836 (KR)
(74) Representative: Roos, Peter
(86) International application number: PCT/KR2021/013992
(87) International publication number: WO 2022/080804

(57) **Abstract**

The present invention relates to: a biomarker composition for predicting the prognosis of brain diseases caused by microplastic exposure; and a use thereof. More particularly, it was confirmed that polyethylene microspheres (PS) in a mouse animal model orally administered with the PS penetrate brain tissue to change the level of gene expression inside the brain tissue, thereby causing brain diseases, and thus the present invention is intended to provide: a biomarker composition for predicting the prognosis of brain diseases caused by microplastic exposure, the biomarker composition using a gene in which the expression level in an individual suspected of exposure to PS is identified; and a method for predicting the prognosis of brain diseases using the biomarker composition.

## Description

### Technical Field

The present disclosure relates to a biomarker composition for predicting prognosis of brain diseases caused by microplastic exposure and a use thereof.

### Background Art

Autism is a neurodevelopmental disorder that impairs the ability to communicate and understand social interactions. The cause of symptom thereof is not yet clear, but some researchers speculate that it is a genetic developmental disorder, not an emotional cause. The incidence rate is 1 in 1000, and the male to female ratio is about 4:1 in the United States. According to a survey conducted in 2011, the prevalence rate of autism in Korea is over 2%, which is drawing attention. Autism is generally referred to as 'autism spectrum disorder (ASD)', which is divided into three categories in academic circles.

First category is autism for children (Kanner's Syndrome) defined by Leo Kanner, second one is Asperger's syndrome as discussed by Hans Asperger, and the last one is high functional autism including savant syndrome.

Growing babies typically show social activities such as staring at people, paying attention to voices, clenching fingers, and smiling. Babies with autism, however, dislike face-to-face contact and have great difficulty in understanding interactions in human society. When it comes to communication, the average babies say words in around their first birthday and show behaviors such as responding to the calling of their names and pointing with fingers at a toy they want, whereas babies with autism choose a different path in their communication development. Some remain silent throughout the life despite sufficient literacy skills but prefer other methods such as drawing or sign language instead.

In relation to the cause of autism, there was a theory that it was acquired in the early stages, but now it is predominant that it is congenital. Brain structure abnormalities, genetic defects, and neurotransmitter abnormalities are usually blamed for the cause. In addition, fetal alcohol syndrome that children are adversely affected by pregnant mothers who like to drink alcohol was also pointed out as a cause. However, the exact causal factor is still controversial.

Although plastic has made great contribution to the affluent daily life of modern people and industrial development owing to excellent functions and low price, it has been reported that the discarded plastic decomposes over time into pieces of microscopic sizes that are difficult to visually identify to be a cause polluting the environment and threatening human health, and the effect of microplastics on brain diseases has not been studied.

### Disclosure of the Invention

### Technical Goals

An object of the present disclosure is to provide a biomarker composition for predicting prognosis of brain diseases caused by microplastic exposure and a method for predicting the prognosis of brain diseases using the same.

### Technical Solutions

The present disclosure provides a biomarker composition for predicting prognosis of a brain disease caused by microplastic exposure, including one or more genes or a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb1bp1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

The present disclosure provides a kit for predicting prognosis of a brain disease caused by microplastic exposure, including a probe specifically binding to one or more genes, a primer for amplifying the one or more genes, antibodies specifically binding to a protein encoded by the one or more genes, or a peptide having a binding domain specific to the protein, wherein the one ore more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

In addition, the present disclosure provides a method for predicting prognosis of a brain disease caused by microplastic exposure, including measuring, from a biological sample isolated from an individual, mRNA expression level of one or more genes or expression level of a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865); and

comparing the mRNA expression level of the one or more genes or the expression level of the protein encoded thereby with a control.

### Advantageous Effects

According to the present disclosure, in a mouse animal model into which polyethylene microspheres (PS) were orally administered, since it was found that PS caused brain diseases by penetrating the brain tissue and changing the gene expression level in the brain tissue, an object of the present disclosure is to provide a biomarker composition for predicting the prognosis of brain diseases caused by microplastic using a gene whose change in the expression level is identified in an individual suspected of PS exposure, and a method for predicting the prognosis of brain diseases using the same.

### Brief Description of Drawings

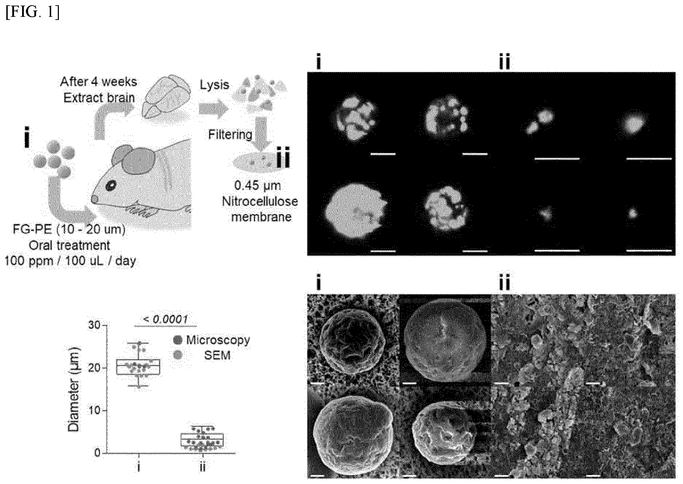

FIG. 1 shows a schematic diagram showing a brain tissue penetration process of orally administered polyethylene microspheres and results of identifying whether polyethylene microspheres penetrate the brain tissue of a mouse animal model into which polyethylene microspheres are orally administered.

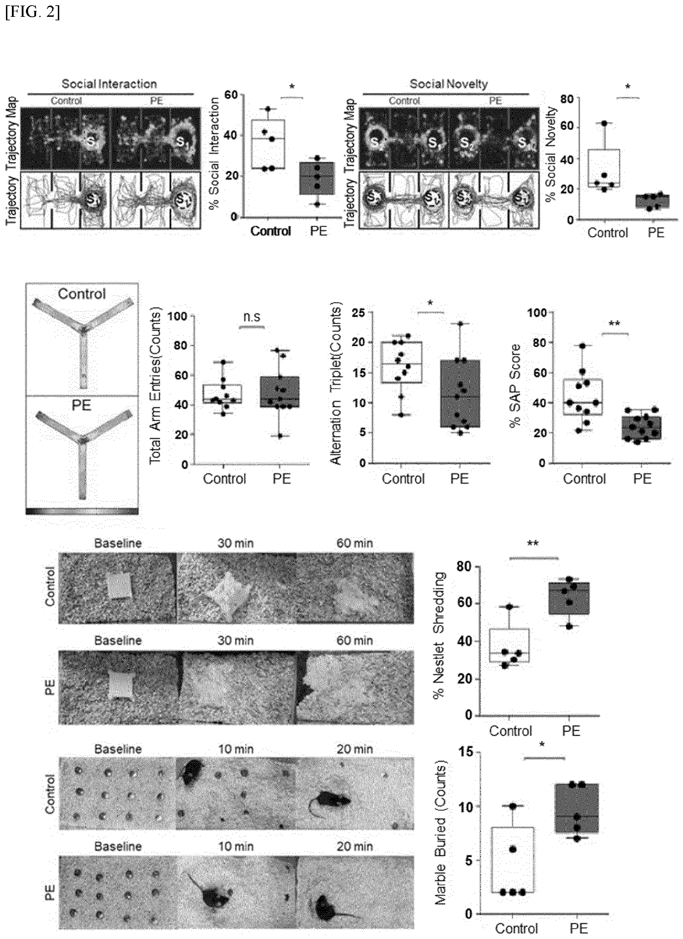

FIG. 2 shows results of performing a 3-chamber experiment (change in sociality), Y-maze (cognition and obsession), marble-burying behavior study (obsessive-compulsive disorder), and nestlet shredding test (obsessive-compulsive disorder) to identify whether autism occurs in a mouse animal model exposed to polyethylene microspheres.

### Best Mode for Carrying Out the Invention

Hereinafter, the present disclosure will be described in more detail.

Since microplastics with a diameter of less than 5 mm are recognized as a new risk factor threatening environment and human health, the inventors of the present disclosure have found, in the course of studying the risk that may exist when polystyrene microspheres (PS), a type of microplastics, are ingested in vivo, that PS absorbed in vivo penetrated into the brain tissue and changed the gene expression level in the brain tissue, such that the inventors completed the present disclosure to provide a biomarker composition for predicting prognosis of brain diseases caused by microplastics using a gene whose change in the expression level is identified.

The present disclosure may provide a biomarker composition for predicting prognosis of a brain disease caused by microplastic exposure, including one or more genes or a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb1bp1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

More specifically, a change in expression level of the one or more genes or protein encoded thereby may be identified in prefrontal cortex by microplastic exposure, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Crm 10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), and Dusp1 (dual specificity phosphatase 1).

In addition, a change in expression level of the one or more genes or protein encoded thereby may be identified in hippocampus by microplastic exposure, wherein the one or more genes are selected from the group consisting of Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb1bp1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

The microplastic exposure may be selected from the group consisting of oral exposure, inhalation exposure, and transdermal exposure.

The microplastic may be selected from the group consisting of polystyrene, polypropylene, polyethylene, polyamide (PA), acrylonitrile-butadiene-styrene (ABS), polytetrafluoroethylene (PTFE), cellulose acetate (CA), polycarbonate (PC), polymethyl methacrylate (PMMA), polyvinyl chloride (PVC), polyethylene terephthalate (PET), acrylic, melamine resin, and polyurethane (PU).

The microplastic may include a harmful substance that is eluted and desorbed from plastics.

The harmful substance eluted from plastics may be selected from the group consisting of mineral oil, styrene dimer, trimer perfluorinated compounds, sterilizing preservatives, polyols, acrylates, phenols, melamine, and nitrosamines.

The harmful substance desorbed from plastics may be selected from the group consisting of heavy metals, sterilizing preservatives, phthalates, bisphenol A, perfluorinated compounds, polychlorinated biphenyls (PCBs), DDTs, hexachlorocyclohexane (HCH), polycyclic aromatic hydrocarbons (PAHs), aliphatic hydrocarbons, nonylphenol, lubricating oil, medicine, carbamazepine (CBZ), 4-methylbenzylidene camphor (4MBC), TriCloSan (TCS), 17α-ethinyl estradiol (EE2), sulfadiazine (SDZ), amoxicillin (AMX), tetracycline (TC), ciprofloxacin (CIP), and trimethoprim (TMP).

The microplastic may include one or more from an additive group that is added to plastic, consisting of plasticizers, flame retardants, stabilizers, antioxidants, UV stabilizers, heat stabilizers, slip agents, lubricants, antistatic agents, curing agents, foaming agents, biocides, water-soluble colorants, organic pigments, inorganic pigments, special effect colorants, fillers, and enhancers.

The brain disease may be selected from the group consisting of schizophrenia, frontal lobe epilepsy, autism, and attention deficit hyperactivity disorder (ADHD).

The present disclosure may provide a kit for predicting prognosis of a brain disease caused by microplastic exposure, including a probe specifically binding to one or more genes, a primer for amplifying the one or more genes, an antibody specifically binding to a protein encoded by the one or more genes, or a peptide having a binding domain specific to the protein, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

The kit may be an RT-PCR kit, a DNA chip kit, or a protein chip kit, but is not limited thereto.

The present disclosure may provide a method for predicting prognosis of a brain disease caused by microplastic exposure, including measuring, from a biological sample isolated from an individual, mRNA expression level of one or more genes or expression level of a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcerig (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb1bp1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865); and

comparing the mRNA expression level of the one or more genes or the expression level of protein encoded thereby with a control.

The measuring of the mRNA expression level may use any one selected from the group consisting of RT-PCR, competitive RT-PCR, real-time RT-PCR, RNase protection assay (RPA), Northern blotting, and DNA chips.

The measuring of the protein expression level may use any one selected from the group consisting of Western blot, enzyme linked immunosorbent assay (ELISA), radioimmunoassay (RIA), radioimmunodiffusion, Ouchterlony immunodiffusion, rocket immunoelectrophoresis, immunohistochemistry, immunoprecipitation assay, complement fixation assay, FACS, and protein chips.

The term "prognosis" as used herein includes determining whether a subject, in other words, a test subject, will have a disease in the future for a specific disease or disorder, or determining the responsiveness of a test subject to the treatment.

### Modes for Carrying Out the Invention

Hereinafter, to help the understanding of the present disclosure, example embodiments will be described in detail. However, the following example embodiments are merely illustrative of the contents of the present disclosure, and the scope of the present disclosure is not limited to the following example embodiments. The example embodiments of the present disclosure are provided to more completely explain the present disclosure to those of ordinary skill in the art.

### <Example 1> Identification of penetration of polyethylene microspheres into a brain tissue upon oral exposure

100 ppm/100 µL of fluorescent polyethylene (PE) microspheres with a diameter of 10-20 µm were orally administered to BALB/c nude mice by 100 µL at a concentration of 100 ppm for 4 weeks.

After 4 weeks, the mice were euthanized, followed by brain removal of the mice. The brain tissue was physically crushed and dried for a day using a freeze dryer, and the dried tissue was sufficiently dissolved in 70% nitric acid (HNO₃) and neutralized using sodium hydroxide (NaOH). After filtering the liquid tissue with a 0.45 um nitrocellulose membrane filter using a vacuum pump, the size and shape of the fluorescent plastic pieces adsorbed onto the filter were observed under a confocal microscope and a scanning electron microscope.

As a result, as shown in FIG. 1, the spherical shape of the PE microspheres was kept intact before administration, but small pieces and irregular shapes were detected on the filter for brain tissue filtration. In addition, the fluorescent PE microspheres were observed to have an average diameter of 20.49 µm before administration, whereas microplastics adsorbed onto the filter having the brain tissue filtrated were observed to have an average diameter of 3 µm. Such the results are evidence to prove that fragmented microplastics penetrated the brain after undergoing the digestion process in the stomach.

From the above results, it was found that microplastics including PE penetrate the brain, and it may be suggested that such the brain penetration of microplastics had an effect on brain diseases including autism.

### <Example 2> Identification of symptoms of autism by polyethylene microsphere exposure

It was reported that social novelty and interaction were reduced in the autism mouse model, it was found that social interaction was reduced in a mouse model induced with autism by transplanting microorganisms of autistic patients, and it was also observed that social interaction was reduced in various other autism-related mouse models.

Accordingly, 100 ppm/100 µL of fluorescent polyethylene (PE) microspheres with a diameter of 10-20 µm were orally administered to C57BL6 mice by 100 µL at a concentration of 100 ppm for 1 week, and to observe whether autism may be induced by PE microsphere exposure, a 3-chamber experiment was performed (change in sociality; Kaidanovich-Beilin, O., et al., Assessment of social interaction behaviors. J Vis Exp, 2011(48)), Y-maze (cognition and obsession; Roullet, F.I. and J.N. Crawley, Mouse models of autism: testing hypotheses about molecular mechanisms. Curr Top Behav Neurosci, 2011. 7: p. 187-212.) and marble-burying behavior study & nestlet shredding test (obsessive-compulsive disorder; Angoa-Perez, M., et al., Marble burying and nestlet shredding as tests of repetitive, compulsive-like behaviors in mice. J Vis Exp, 2013(82): p. 50978.).

Consequently, as shown in FIG. 2, in a result of the 3-chamber experiment, both the social interaction and social novelty of the PE microsphere-exposed mice were reduced compared to the control. In addition, in the result of Y-maze experiment, the alternation triplet and SAP score decreased in PE microsphere-exposed mice.

In addition, as a result of the nest building test, higher % nestlet shredding was observed in mice exposed to PE microspheres, and in the marble-burying behavior experiment, the marble-buried rate in mice exposed to PE microspheres was observed higher.

From the above results, it was determined that exposure to microplastics degraded cognitive function and worsened obsessive-compulsive symptoms.

### <Example 3> Identification of changes in gene expression in the brain region due to microsphere exposure

In the brain region, Egr1 (early growth response protein 1) is known to be involved in methylation, and an increase in Egr1 in the brain region acts as an important factor in regulating oxidative stress. In addition, Arc (activity regulated cytoskeleton associated protein) gene is associated with multiple neuropsychiatric disorders. In particular, the accumulation and increase of the Arc gene in neurons indicate abnormalities in the proteolytic process and are known to be associated with autism. In addition, high level of IL-1 and IL-6 cytokines appears in actual autistic patients.

Accordingly, changes in gene expression in the brain region due to microsphere exposure were identified. 100 ppm/100 µL of PE microspheres were orally administered to BALB/c nude mice for 4 weeks, the mice were euthanized after 4 weeks, and the prefrontal cortex and hippocampus regions of the mouse brain were isolated and removed. After total RNA extraction from each region, microarray analysis was performed.

As a result, as shown in Table 1, changes in various gene expression were identified in mouse brain tissue exposed to PE microspheres. Referring to Table 1, 15 genes showed a decrease and 18 genes an increase in the prefrontal region, while 18 genes showed a decrease and 14 genes an increase in the hippocampal region.

**TABLE 1**

| Prefrontal cortex | | | | |
|---|---|---|---|---|
| Symbol | Full name | Gene ID | FC | P-value |
| Tceanc | transcription elongation factor A N-terminal and central domain containing | 245695 | -1.5 | 0.002 |
| Mx1 | MX dynamin like GTPase 1 | 17857 | -1.56 | 0.002 |
| Ms4a6d | membrane-spanning 4-domains, subfamily A, member 6D | 68774 | -1.75 | 0.003 |
| Arc | activity regulated cytoskeletal-associated protein | 11838 | 1.98 | 0.006 |
| Olfr912 | olfactory receptor 912 | 258806 | -1.87 | 0.007 |
| Casc5 | kinetochore scaffold 1 | 76464 | 1.57 | 0.008 |
| Egr3 | Early growth response 3 | 13655 | 1.53 | 0.009 |
| Gm11565 | predicted gene 11565 | 670550 | 1.55 | 0.01 |
| Gabra6 | gamma-aminobutyric acid (GABA) A receptor, subunit alpha 6 | 14399 | 1.52 | 0.01 |
| Cdkn1a | cyclin-dependent kinase inhibitor 1A (P21) | 12575 | 2.19 | 0.011 |
| Egr1 | early growth response 1 | 13653 | 1.84 | 0.011 |
| Zfp184 | zinc finger protein 184 (Kruppel-like) | 193452 | 1.62 | 0.013 |
| Ms4a14 | membrane-spanning 4-domains, subfamily A, member 14 | 383435 | 1.53 | 0.015 |
| Tas2r121 | taste receptor, type 2, member 121 | 387349 | 1.63 | 0.016 |
| AB124611 | cDNA sequence AB124611 | 382062 | -1.99 | 0.017 |
| Irs4 | insulin receptor substrate 4 | 16370 | 1.5 | 0.019 |
| Fut2 | fucosyltransferase 2 | 14344 | 1.56 | 0.021 |
| Gm10754 | predicted gene 10754 | 100038699 | -1.79 | 0.023 |
| Rgs21 | regulator of G-protein signaling 21 | 624910 | 1.56 | 0.024 |
| Srsx | serine-rich, secreted, X-linked | 100151772 | 1.71 | 0.025 |
| Rad51b | RAD51 paralog B | 19363 | -1.66 | 0.026 |
| Nr4a1 | nuclear receptor subfamily 4, group A, member 1 | 15370 | 1.61 | 0.029 |
| Olfr1442 | olfactory receptor 1442 | 258692 | -1.65 | 0.031 |
| 1700074P13Rik | RIKEN cDNA 1700074P13 gene | 73481 | 1.52 | 0.033 |
| Ctla2a | cytotoxic T lymphocyte-associated protein 2 alpha | 13024 | -1.6 | 0.034 |
| Ifit3b | interferon-induced protein with tetratricopeptide repeats 3B | 667370 | -2.08 | 0.039 |
| Gm4924 | predicted gene 4924 | 237412 | -1.52 | 0.039 |
| Vmn2r95 | vomeronasal 2, receptor 95 | 328759 | -1.52 | 0.04 |
| Trpc7 | transient receptor potential cation channel, subfamily C, member 7 | 26946 | -1.59 | 0.041 |
| Smc4 | structural maintenance of chromosomes 4 | 70099 | -1.51 | 0.042 |
| Sult1c1 | sulfotransferase family, cytosolic, 1C, member 1 | 20888 | 1.53 | 0.043 |
| Tg | thyroglobulin | 21819 | -1.63 | 0.046 |
| Dusp 1 | dual specificity phosphatase 1 | 19252 | 1.65 | 0.048 |

| Hippocampus | | | | |
|---|---|---|---|---|
| Symbol | Full name | Gene ID | FC | P-value |
| Ptpn20 | protein tyrosine phosphatase, non-receptor type 20 | 19256 | 1.51 | 4E-04 |
| Tex14 | testis expressed gene 14 | 83560 | -1.56 | 0.002 |
| Bhlhe23 | basic helix-loop-helix family, member e23 | 140489 | -1.58 | 0.002 |
| Loxl2 | lysyl oxidase like 2 | 94352 | -1.51 | 0.006 |
| Rora | RAR-related orphan receptor alpha | 19883 | 1.51 | 0.007 |
| Nrap | nebulin-related anchoring protein | 18175 | -1.64 | 0.008 |
| Olfr43 5 | olfactory receptor 435 | 258647 | 1.54 | 0.008 |
| Htr2a | 5-hydroxytryptamine (serotonin) receptor 2A | 15558 | 1.53 | 0.008 |
| Fcerig | Fc receptor, IgE, high affinity I, gamma polypeptide | 14127 | -1.55 | 0.014 |
| Ecscr | endothelial cell surface expressed chemotaxis and apoptosis regulator | 68545 | -1.51 | 0.015 |
| Lrrk2 | leucine-rich repeat kinase 2 | 66725 | 1.54 | 0.016 |
| Ddx51 | DEAD box helicase 51 | 69663 | 1.51 | 0.017 |
| Olfr1284 | olfactory receptor 1284 | 258379 | -1.54 | 0.019 |
| Stfa2l1 | stefin A2 like 1 | 268885 | -1.6 | 0.022 |
| Parpbp | PARP1 binding protein | 75317 | -1.53 | 0.022 |
| Drd5 | dopamine receptor D5 | 13492 | 1.52 | 0.022 |
| Olfr1352 | olfactory receptor 1352 | 259074 | 1.71 | 0.024 |
| Olfr1303 | olfactory receptor 1303 | 258397 | 1.53 | 0.024 |
| Eif2ak2 | eukaryotic translation initiation factor 2-alpha kinase 2 | 19106 | -1.57 | 0.024 |
| Eme1 | essential meiotic structure-specific endonuclease 1 | 268465 | 1.51 | 0.026 |
| Pcbd1 | pterin 4 alpha carbinolamine dehydratase/dimerization cofactor of hepatocyte nuclear factor 1 alpha (TCF1) 1 | 13180 | -1.51 | 0.033 |
| Clic1 | chloride intracellular channel 1 | 114584 | -1.53 | 0.038 |
| Itgb1bp1 | integrin beta 1 binding protein 1 | 16413 | 1.54 | 0.041 |
| Serpinb3c | serine (or cysteine) peptidase inhibitor, clade B, member 3C | 381286 | -1.66 | 0.043 |
| BC048671 | cDNA sequence BC048671 | 243535 | -1.51 | 0.044 |
| Zfy2 | zinc finger protein 2, Y-linked | 22768 | 1.61 | 0.047 |
| Gm20865 | predicted gene, 20865 | 100041223 | -1.63 | 0.048 |

In addition, 100 ppm/100 µL of fluorescent polyethylene (PE) microspheres with a diameter of 10-20 µm were orally administered to C57BL6 mice at a concentration of 100 ppm by 100 µL for 1 week, and the mice were euthanized after 1 week to remove the mouse brain. After extracting the total RNA from the removed brain tissue, cDNA was synthesized and to perform qPCR analysis.

As a result, the oxidative stress marker Egr1 increased 2. 13-fold (p<0.05), ARC, a gene associated with the proteolytic process of neurons and the autism, increased 1.97-fold (p<0.05), and IL-6 and IL-1β, genes associated with the inflammatory response and autism, increased 1.85-fold (p<0.05) and 2.64-fold (p<0.01), respectively, compared to the control after exposure to PE microspheres.

From the above results, it was found that brain diseases including autism may be caused by PE microsphere exposure.

As described above, a specific part of the content of the present disclosure is described in detail, for those of ordinary skill in the art, it is clear that the specific description is only a preferred embodiment, and the scope of the present disclosure is not limited thereby. Accordingly, the substantial scope of the present disclosure may be defined by the appended claims and their equivalents.

## Claims

1. A biomarker composition for predicting prognosis of brain diseases caused by microplastic exposure, comprising one or more genes or a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Crm 10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcer1g (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

2. The biomarker composition of claim 1, wherein a change in expression level of the one or more genes or protein encoded thereby is identified in prefrontal cortex by microplastic exposure, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), and Dusp1 (dual specificity phosphatase 1).

3. The biomarker composition of claim 1, wherein a change in expression level of the one or more genes or protein encoded thereby is identified in hippocampus by microplastic exposure, wherein the one or more genes are selected from the group consisting of Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcer1g (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

4. The biomarker composition of claim 1, wherein the microplastic exposure is selected from the group consisting of oral exposure, inhalation exposure, and transdermal exposure.

5. The biomarker composition of claim 1, wherein the microplastic is selected from the group consisting of polystyrene, polypropylene, polyethylene, polyamide (PA), acrylonitrile-butadiene-styrene (ABS), polytetrafluoroethylene (PTFE), cellulose acetate (CA), polycarbonate (PC), polymethyl methacrylate (PMMA), polyvinyl chloride (PVC), polyethylene terephthalate (PET), acrylic, melamine resin, and polyurethane (PU).

6. The biomarker composition of claim 1, wherein the microplastic comprises a harmful substance that is eluted and desorbed from plastics.

7. The biomarker composition of claim 6, wherein the harmful substance eluted from plastics is selected from the group consisting of mineral oil, styrene dimer, trimer perfluorinated compounds, sterilizing preservatives, polyols, acrylates, phenols, melamine, and nitrosamines.

8. The biomarker composition of claim 6, wherein the harmful substance desorbed from plastics is selected from the group consisting of heavy metals, sterilizing preservatives, phthalates, bisphenol A, perfluorinated compounds, polychlorinated biphenyls (PCBs), DDTs, hexachlorocyclohexane (HCH), polycyclic aromatic hydrocarbons (PAHs), aliphatic hydrocarbons, nonylphenol, lubricating oil, medicine, carbamazepine (CBZ), 4-methylbenzylidene camphor (4MBC), TriCloSan (TCS), 17α-ethinyl estradiol (EE2), sulfadiazine (SDZ), amoxicillin (AMX), tetracycline (TC), ciprofloxacin (CIP), and trimethoprim (TMP).

9. The biomarker composition of claim 1, wherein the microplastic comprises one or more from an additive group that is added to the plastic, consisting of plasticizers, flame retardants, stabilizers, antioxidants, UV stabilizers, heat stabilizers, slip agents, lubricants, antistatic agents, curing agents, foaming agents, biocides, water-soluble colorants, organic pigments, inorganic pigments, special effect colorants, fillers, and enhancers.

10. The biomarker composition of claim 1, wherein the brain disease is selected from the group consisting of schizophrenia, frontal lobe epilepsy, autism, and attention deficit hyperactivity disorder (ADHD).

11. A kit for predicting prognosis of a brain disease caused by microplastic exposure, comprising a probe specifically binding to one or more genes, a primer for amplifying the one or more genes, an antibody specifically binding to a protein encoded by the one or more genes, or a peptide having a binding domain specific to the protein, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcer1g (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865).

12. The kit of claim 11, wherein the kit is an RT-PCR kit, a DNA chip kit, or a protein chip kit.

13. A method for predicting prognosis of a brain disease caused by microplastic exposure, comprising:
measuring, from a biological sample isolated from an individual, mRNA expression level of one or more genes or expression level of a protein encoded thereby, wherein the one or more genes are selected from the group consisting of Tceanc (transcription elongation factor A N-terminal and central domain containing), Mx1 (MX dynamin like GTPase 1), Ms4a6d (membrane-spanning 4-domains, subfamily A, member 6D), Arc (activity regulated cytoskeletal-associated protein), Olfr912 (olfactory receptor 912), Casc5 (kinetochore scaffold 1), Egr3 (Early growth response 3), Gm11565 (predicted gene 11565), Gabra6 (gamma-aminobutyric acid (GABA) A receptor, subunit alpha 6), Cdkn1a (cyclin-dependent kinase inhibitor 1A (P21)), Egr1 (early growth response 1), Zfp184 (zinc finger protein 184 (Kruppel-like)), Ms4a14 (membrane-spanning 4-domains, subfamily A, member 14), Tas2r121 (taste receptor, type 2, member 121), AB124611 (cDNA sequence AB124611), Irs4 (insulin receptor substrate 4), Fut2 (fucosyltransferase 2), Gm10754 (predicted gene 10754), Rgs21 (regulator of G-protein signalling 21), Srsx (serine-rich, secreted, X-linked), Rad51b (RAD51 paralog B), Nr4a1 (nuclear receptor subfamily 4, group A, member 1), Olfr1442 (olfactory receptor 1442), 1700074P13Rik (RIKEN cDNA 1700074P13 gene), Ctla2a (cytotoxic T lymphocyte-associated protein 2 alpha), Ifit3b (interferon-induced protein with tetratricopeptide repeats 3B), Gm4924 (predicted gene 4924), Vmn2r95 (vomeronasal 2, receptor 95), Trpc7 (transient receptor potential cation channel, subfamily C, member 7), Smc4 (structural maintenance of chromosomes 4), Sult1c1 (sulfotransferase family, cytosolic, 1C, member 1), Tg (thyroglobulin), Dusp1 (dual specificity phosphatase 1), Ptpn20 (protein tyrosine phosphatase, non-receptor type 20), Tex14 (testis expressed gene 14), Bhlhe23 (basic helix-loop-helix family, member e23), Loxl2 (lysyl oxidase like 2), Rora (RAR-related orphan receptor alpha), Nrap (nebulin-related anchoring protein), Olfr435 (olfactory receptor 435), Htr2a (5-hydroxytryptamine (serotonin) receptor 2A), Fcer1g (Fc receptor, IgE, high affinity I, gamma polypeptide), Ecscr (endothelial cell surface expressed chemotaxis and apoptosis regulator), Lrrk2 (leucine-rich repeat kinase 2), Ddx51 (DEAD box helicase 51), Olfr1284 (olfactory receptor 1284), Stfa2l1 (stefin A2 like 1), Parpbp (PARP1 binding protein), Drd5 (dopamine receptor D5), Olfr1352 (olfactory receptor 1352), Olfr1303 (olfactory receptor 1303), Eif2ak2 (eukaryotic translation initiation factor 2-alpha kinase 2), Eme1 (essential meiotic structure-specific endonuclease 1), Pcbd1 (pterin 4 alpha carbinolamine dehydratase/dimerization cofactor of hepatocyte nuclear factor 1 alpha (TCF1) 1), Clic1 (chloride intracellular channel 1), Itgb 1 bp 1 (integrin beta 1 binding protein 1), Serpinb3c (serine (or cysteine) peptidase inhibitor, clade B, member 3C), BC048671 (cDNA sequence BC048671), Zfy2 (zinc finger protein 2, Y-linked), and Gm20865 (predicted gene, 20865); and
comparing the mRNA expression level of the one or more genes or the expression level of the protein encoded thereby with a control.

14. The method of claim 13, wherein the measuring of the mRNA expression level uses any one selected from the group consisting of RT-PCR, competitive RT-PCR, real-time RT-PCR, RNase protection assay (RPA), Northern blotting, and DNA chips.

15. The method of claim 13, wherein the measuring of the protein expression level uses any one selected from the group consisting of Western blot, enzyme linked immunosorbent assay (ELISA), radioimmunoassay (RIA), radioimmunodiffusion, Ouchterlony immunodiffusion, rocket immunoelectrophoresis, immunohistochemistry, immunoprecipitation assay, complement fixation assay, FACS, and protein chips.
